# FASCICULE DE BREVET EUROPEEN

(11) **EP 0 901 364 B1**
(45) Date de publication et mention de la délivrance du brevet: **25.04.2001**
(21) Numéro de dépôt: 97925107.1
(22) Date de dépôt: 20.05.1997
(51) Int. Cl.: A61K 7/13

(54) **COMPOSITION DE TEINTURE DIRECTE CAPILLAIRE COMPRENANT UN POLYMERE RETICULE A MOTIFS ACRYLIQUES ET/OU ACRYLATES ET A MOTIFS ACRYLAMIDES**
ZUSAMMENSETZUNG ZUR DIREKTFAERBUNG VON HAAREN, DIE EIN VERNETZTES POLYMER MIT ACRYLEINHEITEN UND/ODER ACRYLATEINHEITEN UND MIT ACRYLAMIDEINHEITEN ENTHAELT
DIRECT CAPILLARY DYEING COMPOSITION COMPRISING A CROSS-LINKED POLYMER WITH ACRYLIC AND/OR ACRYLATE UNITS AND WITH ACRYLAMIDE UNITS

(30) Priorité: 23.05.1996 FR 9606429
(43) Date de publication de la demande: 17.03.1999
(73) Titulaire: L'OREAL, 75008 Paris (FR)
(72) Inventeur: MAUBRU, Mireille, F-78400 Chatou (FR)
(74) Mandataire: Andral, Christophe André Louis
(86) Numéro de dépôt international: FR9700884
(87) Numéro de publication internationale: WO9744003

(56) Documents cités:
- EP-A- 0 445 714
- EP-A- 0 503 507
- DE-A- 3 044 754
- DE-U- 9 413 897

## Description

L'invention concerne une composition de teinture pour cheveux comprenant au moins un colorant direct et au moins un polymère réticulé à motifs acryliques et/ou acrylates et à motifs acrylamides.

Il est connu de teindre les fibres capillaires avec des compositions de teinture directe suivant un procédé dit de «coloration directe» qui consiste à appliquer sur les fibres, des molécules colorantes ayant une affinité pour lesdites fibres, à les laisser pauser puis à rincer les fibres. Les colorations qui en résultent sont des colorations temporaires ou semi-permanentes suivant la nature des interactions entre les colorants directs et la fibre capillaire, et leur désorption de la surface et/ou du coeur de la fibre.
Pour faciliter l'application de telles compositions de teinture sur les cheveux, éviter notamment qu'elles ne coulent sur le front et le visage ou en dehors du point d'application initialement choisi, pendant l'application ou au cours du temps de pause nécesssaire à la coloration, on augmente classiquement la viscosité des compositions au moyen d'acide polyacrylique réticulé (agent épaississant).
Cependant les compositions de teinture à base de colorants directs et d'acide polyacrylique réticulé ne s'avèrent plus suffisamment satisfaisantes au niveau de leurs propriétés tinctoriales après qu'elles ont été stockées un certain temps à une température plus basse que la température ambiante, par exemple en dessous de 10°C, et notamment aux environs de 4°C.
On observe ainsi que les compositions stockées dans de telles conditions engendrent une montée plus faible du colorant direct sur les cheveux et présentent donc un pouvoir tinctorial insuffisant.

La présente invention vise à résoudre le problème ci-dessus, c'est-à-dire à proposer un moyen permettant de préserver le pouvoir tinctorial de compositions de teinture renfermant un colorant direct, susceptibles d'être stockées à basses températures, en particulier à des températures inférieures à 10°C.

Or, après de nombreuses recherches menées sur la question, la demanderesse vient maintenant de découvrir, qu'il est possible de préserver le pouvoir tinctorial des compositions de coloration directe, si l'on ajoute à ces compositions, une quantité efficace d'un polymère réticulé à motifs acryliques et/ou acrylates et à motifs acrylamides.

Même après des conservations plus ou moins prolongées à des températures inférieures à 10°C, et notamment voisines de 4°C, on obtient des compositions présentant un bon pouvoir tinctorial, et dont la montée sur les cheveux est très satisfaisante.

Cette découverte est à la base de la présente invention.

La présente invention a ainsi pour objet une composition cosmétique de teinture capillaire, du type comprenant, dans un support cosmétiquement acceptable approprié pour la teinture, au moins un colorant direct, et qui est caractérisée par le fait qu'elle comprend en outre au moins un polymère réticulé à motifs acryliques et/ou acrylates et à motifs acrylamides.

La présente invention a également pour objet l'utilisation d'un polymère réticulé à motifs acryliques et/ou acrylates et à motifs acrylamides dans ou pour la fabrication d'une, composition de teinture directe pour cheveux comprenant au moins un colorant direct, pour améliorer la conservation du pouvoir tinctorial de ladite composition, en particulier après des stockages en dessous de 10°C environ, et notamment aux environs de 4°C.

L'invention concerne aussi un procédé pour améliorer la conservation du pouvoir tinctorial, en particulier après des stockages en dessous de 10°C environ, et notamment aux environs de 4°C, d'une composition de teinture pour cheveux comprenant au moins un colorant direct, qui consiste à introduire dans ladite composition, une quantité efficace d'au moins un polymère réticulé à motifs acryliques et/ou acrylates et à motifs acrylamides.

Elle concerne enfin un procédé de teinture capillaire mettant en oeuvre les compositions à propriétés améliorées conformes à l'invention.

Mais d'autres caractéristiques, aspects, objets et avantages de l'invention apparaîtront encore plus clairement à la lecture de la description et des exemples qui suivent.

Selon l'invention, on entend désigner par motifs acryliques et/ou acrylates des motifs de structure : 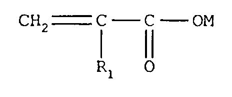 dans laquelle,
- R₁ désigne H ou CH₃, de préférence H,
- M désigne l'hydrogène, un ammonium, un métal alcalin tel que le sodium ou le potassium, ou une monoamine primaire ou secondaire, et de préférence un ammonium.
   On entend également désigner par motifs acrylamides des motifs de structure : 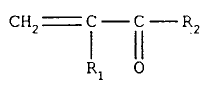 dans laquelle,
- R₁ désigne H ou CH₃, de préférence H,
- R₂ désigne un radical amino, diméthylamino, tert-butylamino ou -NH-CH₂OH, et de préférence un radical amino.

De préférence, les polymères utilisés dans le cadre de la présente invention sont des polymères du type à motifs acrylates (M différent de H) et à motifs acrylamides, réticulés au moyen d'un agent réticulant approprié.

Le ou les polymère(s) réticulé(s) à motifs acryliques et/ou acrylates et à motifs acrylamides utilisable(s) dans le cadre de la présente invention, sont ainsi plus particulièrement des copolymères acrylate d'ammonium/acrylamide ou acrylate de sodium/acrylamide réticulés par un composé à polyinsaturation oléfinique, de préférence choisi parmi le divinylbenzène, le tétraallyloxyéthane, le méthylènebisacrylamide, l'éther diallylique, les éthers polyallylpolyglycéryliques ou les éthers allyliques d'alcools de la série des sucres, tels que erythritol, pentaerythritol, arabitol, mannitol, sorbitol ou glucose.
Des copolymères réticulés de ce type sont décrits et préparés dans le brevet français FR-2 416 723 et les brevets US-2 798 053 et US-2 923 692.

On préfère selon l'invention tout particulièrement utiliser des copolymères réticulés du type acrylate d'ammonium/acrylamide, et de façon plus préférentielle encore un copolymère de ce type 95/5 en poids, sous la forme d'une émulsion eau-dans-huile, constituée de 32% en poids dudit copolymère, 20% en poids d'isoparaffine C₁₁-C₁₃, 2% en poids de sesquioléate de sorbitan, de 3% en poids d'un dérivé éthoxylé hydrophile, et 43% en poids d'eau. Une telle émulsion est commercialisée par la société HOECHST sous la dénomination BOZEPOLE C NOUVEAU.

Le polymère réticulé à motifs acryliques et/ou acrylates et à motifs acrylamides est généralement utilisé dans la composition tinctoriale selon l'invention dans des proportions en matière active pouvant aller d'environ 0,05 à environ 5%, et de préférence d'environ 0,1 à environ 3% en poids par rapport au poids total de la composition.

Les colorants directs utilisables dans la composition tinctoriale selon la présente invention sont les colorants directs au sens défini ci-avant, c'est-à-dire utilisables suivant un procédé classique de coloration directe.

Parmi ceux classiquement utilisés, on peut citer des colorants nitrés benzéniques, tels que les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques ou encore des colorants métallifères.

Les colorants directs plus particulièrement préférés selon l'invention sont choisis parmi les suivants :
**i)** les colorants nitrés benzéniques de formule (I) suivante : 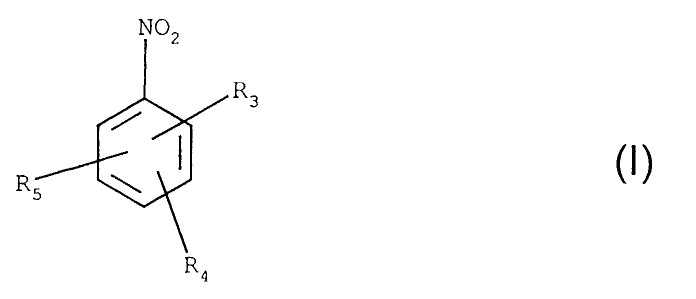 dans laquelle :
   - **R**_{**3**} désigne un radical NH₂, amino monosubstitué par un radical alkyle, monohydroxyalkyle, polyhydroxyalkyle, aminoalkyle, ou amino disubstitué par des radicaux, identiques ou différents, alkyle, mono- ou poly-hydroxyalkyle, ou aminoalkyle,
   - **R**_{**4**} désigne hydrogène, hydroxy, alcoxy, mono- ou poly-hydroxyalkyloxy, ou les mêmes significations désignées ci-dessus pour R₃, à l'exception du radical amino disubstitué,
   - **R**_{**5**} désigne hydrogène, alkyle, nitro, ou halogène,
**ii)** les colorants anthraquinoniques de formule (II) suivante : 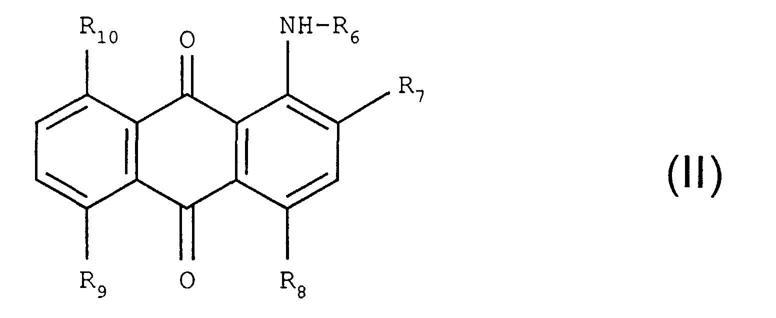 dans laquelle,
   - **R**_{**6**} désigne hydrogène, un radical monohydroxyalkyle ou polyhydroxyalkyle,
   - **R**_{**7**} désigne hydrogène, un radical alkyle ou alcoxy,
   - **R**_{**8**} désigne hydrogène, un radical hydroxy, amino ou monohydroxyalkylamino ou polyhydroxyalkylamino,
   - **R**_{**9**} et **R**_{**10**}, identiques ou différents sont hydrogène, hydroxy ou amino,
**iii)** les colorants azoïques de formule (III) suivante : 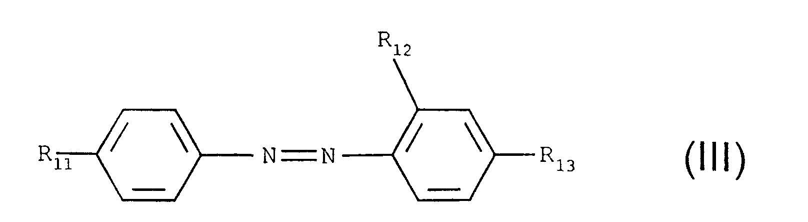 dans laquelle :
   - **R**_{**11**} désigne un radical nitro, amino, amino mono- ou di-substitué par des alkyles,
   - **R**_{**12**} désigne hydrogène ou un radical alkyle,
   - **R**_{**13**} désigne un radical amino, amino mono- ou di-substitué par des monohydroxyalkyles, étant entendu que les radicaux alkyles et alcoxy cités ci-avant dans les formules (I), (II) et (III) sont en C₁-C₄ et qu'ils peuvent être linéaires ou ramifiés, et les sels cosmétiquement acceptables de tous ces composés.

Par C₁-C₄, on entend notamment les radicaux méthyle, éthyle, n-propyle, isopropyle, n-butyle, sec-butyle, et tert-butyle.
Par sels cosmétiquement acceptables, on désigne plus particulièrement les chlorhydrates, bromhydrates, et les sulfates.

Plus avantageusement encore, selon la présente invention, on préfère mettre en oeuvre les colorants directs suivants :
- 1-amino-2-nitro-4-N-(β-hydroxyéthyl)-amino-5-méthyl-benzène,
- 1,4,5,8-tétraaminoanthraquinone,
- 1,4-bis-N,N'-[(β,γ-dihydroxypropyl)-amino]-anthraquinone
- 1,4,4-N-tris-(β-hydroxyéthyl)-1,4-diamino-2-nitro-benzène,
- 1-N-(β-hydroxyéthyl)-amino-2-nitro-4-amino-benzène,
- 1-hydroxy-3-nitro-4-amino-benzène,
- 1-hydroxy-3-nitro-4-N-(β-hydroxyéthyl)-amino-benzène,
- 1-β-hydroxyéthyloxy-3-méthylamino-4-nitro-benzène,
- 1-méthylamino-2-nitro-5-β,γ-dihydroxypropyloxy-benzène,
- 1-N-(β-aminoéthyl)-amino-2-nitro-4-β-hydroxyéthyloxy-benzène,
- 4-[N-éthyl-N-(β-hydroxyéthyl)-amino]-1-N-(β-hydroxyéthyl)-amino-2-nitrobenzène,
- 1-(4'-amino-diphénylazo)-2-méthyl-4-N-bis-(β-hydroxyéthyl)-amino-benzène,
- 1-méthoxy-3-N-(β-aminoéthyl)-amino-4-nitro-benzène,
- 1-amino-2-nitro-4-N-(β-hydroxyéthyl)-amino-benzène,
- 1-amino-2-nitro-4-N-bis-(β-hydroxyéthyl)-amino-benzène,
- 1,4-N-bis-(β-hydroxyéthyl)-amino-2-nitro-benzène,
- 1-amino-2-N-(β-hydroxyéthyl)-amino-5-nitro-benzène,
- 1,4-diamino-anthraquinone,
et leurs sels cosmétiquement acceptables.

Ces colorants directs, sous forme de base ou salifiée, sont généralement présents dans la composition tinctoriale selon l'invention dans des proportions pouvant aller d'environ 0,001 à environ 10%, et de préférence d'environ 0,05 à environ 5% en poids par rapport au poids total de la composition.

Le milieu cosmétiquement acceptable pour la teinture est un milieu aqueux pouvant contenir un ou plusieurs solvants organiques choisis par exemple parmi l'alcool éthylique, l'alcool isopropylique, l'alcool benzylique et l'alcool phényléthylique, ou des glycols ou éthers de glycol, tels que par exemple, le propylèneglycol ou ses éthers tels que, par exemple, le monométhyléther de propylèneglycol, le butylèneglycol, le dipropylèneglycol, les éthers monométhylique, monoéthylique et monobutylique de l'éthylèneglycol, ainsi que les alkyléthers de diéthylèneglycol, comme par exemple, le monoéthyléther ou le monobutyléther du diéthylèneglycol, dans des proportions comprises entre environ 0,5 et 20% et, de préférence, entre environ 2 et 10% en poids par rapport au poids total de la composition.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
Ladite composition tinctoriale peut en outre contenir divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des fibres kératiniques humaines et notamment des cheveux.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 3 à 12 et de préférence de 7 à 11 et plus préférentiellement encore de 8,5 à 10, et pouvant être ajusté au moyen d'agents d'alcalinisation ou d'agents d'acidification antérieurement bien connus.
Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 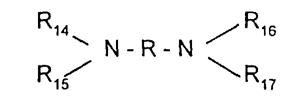 dans laquelle, R est un reste propylène éventuellement substitué par un groupement hydroxyle ou un radical alkyle en C₁-C₄ ; R₁₄ R₁₅, R₁₆ et R₁₇, simultanément ou indépendamment l'un de l'autre, représentent un atome d'hydrogène, un radical alkyle en C₁-C₄ ou hydroxyalkyle en C₁-C₄.
Les agents acidifiants sont classiquement des acides minéraux ou organiques comme par exemple les acides chlorhydrique, tartrique, citrique et phosphorique.

La composition appliquée sur les cheveux peut se présenter sous des formes diverses, telles que sous forme de liquide, de crème, de gel ou sous toute autre forme appropriée pour réaliser une teinture des cheveux. En particulier, elle peut être conditionnée sous pression en flacon aérosol en présence d'un agent propulseur et former une mousse.

Un autre objet de la présente invention porte sur un procédé de teinture des cheveux, par coloration directe, consistant à appliquer sur les cheveux secs ou humides, une composition tinctoriale telle que définie ci-avant, puis à laisser agir ladite composition de préférence pendant 3 à 60 minutes environ, à rincer les cheveux, puis à les laver éventuellement, à les rincer ensuite à nouveau, puis à les sécher.
On peut aussi laisser agir la composition, puis la sécher.

Des exemples concrets illustrant l'invention vont maintenant être donnés.

### EXEMPLE 1 :

On a préparé la composition de teinture suivante :

| | |
|---|---|
| Colorant direct (1)* | 0,1 g |
| Alcool décylique oxyéthyléné par 5,3 moles d'oxyde d'éthylène | 2,0 g |
| Acide laurique | 1,0 g |
| Monobutyléther du diéthylèneglycol | 5,0 g |
| BOZEPOLE C NOUVEAU de Hoechst | 0,9 g MA^{•} |
| 2-amino-2-méthyl-1-propanol q.s pH | 9,5 |
| Eau déminéralisée q.s.p. | 100 g |

| | |
|---|---|
| *colorant (1) : 1-amino-2-nitro-4-N-(β-hydroxyéthyl)amino-5 -méthyl-benzène. MA^{•} désigne Matière Active. | |

Après 24 heures, on a mesuré la viscosité de cette composition au viscosimètre Contrave à 25°C. La viscosité enregistrée était de 210 cp.
On a ensuite appliqué cette composition sur des mèches de cheveux gris naturels à 90% de blancs, et on a laissé pauser la composition pendant 30 minutes. Puis on a rincé les mèches à l'eau courante et on les a séchées.
Les mèches ont été teintes dans une nuance, qui chiffrée en valeur MUNSELL (Norme ASTM D 1535-68, laquelle définit la couleur : H, désignant la nuance ou Hue, V, désignant l'intensité ou Value, et C, désignant la pureté ou Chromaticité), sur un colorimètre MINOLTA CM 2002, était la suivante :
en H,V,C : **6,9 R 4,7 / 3,0.**
Les mèches témoin (non teintes) présentaient une nuance H,V,C : **3,8Y 5,7 / 1,6.**

On a également stocké la composition ci-dessus pendant 1 mois à la température de 4°C.
Puis on a appliqué la composition ainsi conservée sur des mèches de cheveux de même qualité et suivant le même protocole que ci-dessus.
La nuance des mèches teintes au moyen de cette composition conservée à 4°C était la suivante :
en H,V,C : **7,9 R 4,7 / 3,0.**
Puis on a quantifié la modification de couleur entre les mèches teintes au moyen de la composition initiale et celles teintes au moyen de la composition conservée durant 1 mois, à la température de 4°C, en utilisant l'équation de NICKERSON qui définit les indices de variation de couleur :
**I=** (C/5) x 2ΔH + 6ΔV + 3ΔC (cette équation étant décrite dans la publication : «Journal of the Optical Society of America», 1944-sept-Vo134-n°9-p550-570).
Ainsi la modification de coloration **I**_{**b**} (indice de variation de couleur entre des mèches teintes au moyen de la composition conservée 1 mois à la température de 4°C et celle des mèches teintes au moyen de la composition initiale) rapportée à **I**_{**a**} (indice de variation de couleur des mèches teintes au moyen de la composition initiale et celle des mèches témoin), chiffrée en %, a été de 5,7%.

### EXEMPLE 2 COMPARATIF :

On a préparé une composition de teinture semblable à celle de l'exemple 1, de viscosité égale à celle de l'exemple 1, à base de polymère de l'art antérieur, en remplaçant simplement les 0,9 g de copolymère réticulé acrylate d'ammonium/acrylamide (BOZEPOLE C NOUVEAU) par 0,57 g de CARBOPOL 980 de la société Goodrich (acide polyacrylique réticulé de l'art antérieur-PM 4 000 000).
Des mèches de cheveux naturels à 90% de blancs ont été teintes au moyen de la composition initiale (i.e. avant conservation) et suivant un protocole identique à celui de l'exemple 1 en une nuance exprimée en H,V,C égale à : **8,1 R 4,9 / 2,9.**
Des mèches de cheveux naturels à 90% de blancs ont été teintes au moyen de la même composition mais conservée 1 mois à 4°C. La nuance obtenue a été :
H,V,C : **8,7 R 4,8 / 2,8.**
Le rapport **I**_{**b**} **/ I**_{**a**} appliqué à cet exemple, et chiffré en %, a été de 8,5%.

### Conclusion:

Après conservation pendant 1 mois à 4°C, la composition de teinture de l'exemple 1 comprenant un polymère réticulé conforme à la présente invention, présente un pouvoir tinctorial très supérieur à celui de la composition de teinture de l'exemple 2 comprenant un polymère réticulé de l'art antérieur, puisque la dégradation qui est exprimée par le rapport **I**_{b} / **I**ₐ chiffré en %, n'est que de 5,7% dans le cas de l'exemple 1, alors qu'elle est de 8,5% dans le cas de l'exemple 2.

### EXEMPLE 3 :

On a préparé la composition de teinture suivante :

| | |
|---|---|
| Colorant direct (2)* | 0,1 g |
| Alcool décylique oxyéthyléné par 5,3 moles d'oxyde d'éthylène | 2,0 g |
| Acide laurique | 1,0 g |
| Monobutyléther du diéthylèneglycol | 5,0 g |
| BOZEPOLE C NOUVEAU de Hoechst | 0,57g MA^{•} |
| 2-amino-2-méthyl-1-propanol q.s pH | 9,5 |
| Eau deminéralisée q.s.p | 100 g |

| | |
|---|---|
| *colorant (2) : 1,4,5,8-tétraaminoanthraquinone (dispersée à 30% sur lignosulfate). | |

Après 24 heures, on a mesuré la viscosité de cette composition au viscosimètre Contrave à 25°C. La viscosité enregistrée était de 190 cp.
On a ensuite appliqué cette composition sur des mèches de cheveux gris permanentés à 90% de blancs, et on a laissé pauser la composition pendant 30 minutes. Puis on a rincé les mèches à l'eau courante et on les a séchées.
Les mèches ont été teintes dans une nuance, qui chiffrée en valeur MUNSELL, était la suivante, en H,V,C : **4,4 B 4,0 / 2,4**.
Les mèches témoin (non teintes) présentaient une nuance H,V,C : **4,4 Y 5,9 / 1,6.**

On a ensuite conservé ladite composition ci-dessus pendant 1 mois à la température de 4°C.
Puis on a appliqué la composition ainsi conservée sur des mèches de cheveux de même qualité et suivant le même protocole que ci-dessus.
La nuance des mèches teintes au moyen de cette composition conservée à 4°C était la suivante, en H,V,C : **4,7 B 4,2/2,4.**
Le rapport **I**_{b} (indice de variation de couleur entre des mèches teintes au moyen de la composition conservée 1 mois à la température de 4°C et celle des mèches teintes au moyen de la composition initiale) à **I**_{**a**} (indice de variation de couleur entre des mèches teintes au moyen de la composition initiale et celle des mèches témoin), chiffré en %, a été de 3,8%.

### EXEMPLE 4 COMPARATIF :

On a préparé une composition de teinture semblable à celle de l'exemple 3, de viscosité égale à celle de l'exemple 3, à base de polymère de l'art antérieur, en remplaçant simplement les 0,57 g de copolymère réticulé acrylate d'ammonium/acrylamide (BOZEPOLE C NOUVEAU) par 0,67 g de CARBOPOL 2984 de la société Goodrich (acide polyacrylique réticulé de l'art antérieur-PM 3 000 000).
Des mèches de cheveux permanentés à 90% de blancs ont été teintes au moyen de la composition initiale (i.e. avant conservation) et suivant un protocole identique à celui de l'exemple 3 en une nuance exprimée en H,V,C égale à : **5,4 B 4,1 / 3,1.**
Des mèches de cheveux permanentés à 90% de blancs ont été teintes au moyen de la même composition mais conservée 1 mois à 4°C. La nuance obtenue a été égale en H,V,C, à : **1,6 B 4,3 / 1,9.**
Le rapport **I**_{**b**} **/ I**_{**a**} appliqué à cet exemple, et chiffré en %, a été de 22,9%.

### Conclusion :

Après conservation pendant 1 mois à 4°C, la composition de teinture de l'exemple 3 comprenant un polymère réticulé conforme à la présente invention, présente un pouvoir tinctorial très supérieur à celui de la composition de teinture de l'exemple 4 comprenant un polymère réticulé de l'art antérieur, puisque la dégradation qui est exprimée par le rapport **I**_{**b**} / **I**_{**a**} chiffré en %, n'est que de 3,8% dans le cas de l'exemple 3, alors qu'elle est de 22,9% dans le cas de l'exemple 4.

## Revendications

1. Composition de teinture capillaire, du type comprenant, dans un support cosmétiquement acceptable approprié pour la teinture, au moins un colorant direct, caractérisée par le fait qu'elle comprend en outre au moins un polymère réticulé à motifs acryliques et/ou acrylates et à motifs acrylamides.

2. Composition de teinture selon la revendication 1, caractérisée par le fait que le polymère réticulé est à motifs acrylates et à motifs acrylamides.

3. Composition de teinture selon la revendication 2, caractérisée par le fait que le polymère réticulé est un copolymère réticulé acrylate d'ammonium/acrylamide.

4. Composition de teinture selon la revendication 2, caractérisée par le fait que le polymère réticulé est un copolymère réticulé acrylate de sodium/acrylamide.

5. Composition de teinture selon l'une quelconque des revendications précédentes, caractérisée par le fait que l'agent de réticulation est un composé à polyinsaturation oléfinique choisi parmi le divinylbenzène, le tétraallyloxyéthane, le méthylènebis-acrylamide, l'éther diallylique, les éthers polyallylpolyglycéryliques ou les éthers allyliques d'alcools de la série des sucres.

6. Composition selon les revendications 1, 2, 3 et 5, caractérisée par le fait que le polymère réticulé est un copolymère réticulé acrylate d'ammonium/acrylamide à raison de 95/5 en poids.

7. Composition selon la revendication 6, caractérisée par le fait que le copolymère est sous forme d'une émulsion eau-dans-huile, constituée de 32% en poids dudit copolymère, 20% en poids d'isoparaffine C₁₁-C₁₃, 2% en poids de sesquioléate de sorbitan, 3% en poids d'un dérivé éthoxylé hydrophile, et 43% en poids d'eau.

8. Composition selon l'une quelconque des revendications précédentes, caractérisée par le fait que le colorant direct est un colorant nitré benzénique de formule (I) suivante : 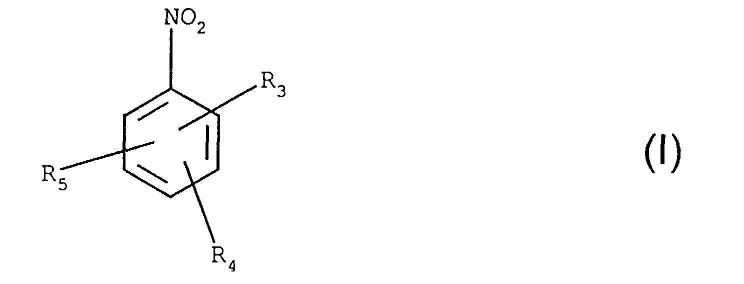 dans laquelle :
- **R**_{**3**} désigne un radical NH₂, amino monosubstitué par un radical alkyle, monohydroxyalkyle, polyhydroxyalkyle, aminoalkyle, ou amino disubstitué par des radicaux, identiques ou différents, alkyle, mono- ou poly-hydroxyalkyle, ou aminoalkyle,
- **R**_{**4**} désigne hydrogène, hydroxy, alcoxy, mono- ou poly-hydroxyalkyloxy, ou les mêmes significations désignées ci-dessus pour R₃, à l'exception du radical amino disubstitué,
- **R**_{**5**} désigne hydrogène, alkyle, nitro, ou halogène,
étant entendu que les radicaux alkyles et alcoxy cités ci-avant sont en C₁-C₄ et qu'ils peuvent être linéaires ou ramifiés,
et les sels cosmétiquement acceptables de ces composés.

9. Composition selon les revendications 1 à 7, caractérisée par le fait que le colorant direct est un colorant anthraquinonique de formule (II) suivante : 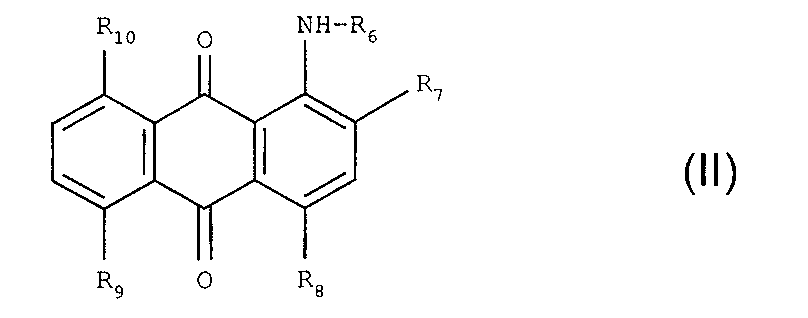 dans laquelle,
- **R**_{**6**} désigne hydrogène, un radical monohydroxyalkyle ou polyhydroxyalkyle,
- **R**_{**7**} désigne hydrogène, un radical alkyle ou alcoxy,
- **R**_{**8**} désigne hydrogène, un radical hydroxy, amino ou monohydroxyalkylamino ou polyhydroxyalkylamino,
- **R**_{**9**} et **R**_{**10**}, identiques ou différents sont hydrogène, hydroxy ou amino,
étant entendu que les radicaux alkyles et alcoxy cités ci-avant sont en C₁-C₄ et qu'ils peuvent être linéaires ou ramifiés,
et les sels cosmétiquement acceptables de ces composés.

10. Composition selon les revendications 1 à 7, caractérisée par le fait que le colorant direct est un colorant azoïque de formule (III) suivante : 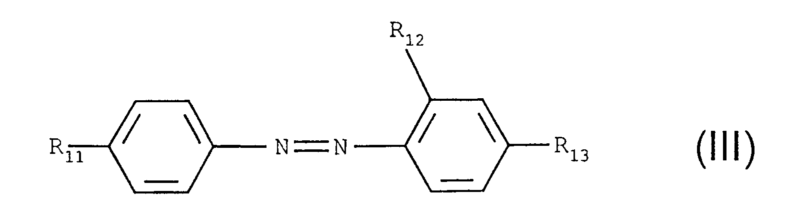 dans laquelle :
- **R**_{**11**} désigne un radical nitro, amino, amino mono- ou di-substitué par des alkyles,
- **R**_{**12**} désigne hydrogène ou un radical alkyle,
- **R**_{**13**} désigne un radical amino, amino mono- ou di-substitué par des monohydroxyalkyles,
étant entendu que les radicaux alkyles et alcoxy cités ci-avant sont en C₁-C₄ et qu'ils peuvent être linéaires ou ramifiés,
et les sels cosmétiquement acceptables de ces composés.

11. Composition selon les revendications 8 à 10, caractérisée par le fait que les sels cosmétiquement acceptables sont des chlorhydrates, des bromhydrates et des sulfates.

12. Composition selon l'une quelconque des revendications précédentes, caractérisée par le fait que polymère réticulé est présent, calculé en matière active, dans des proportions allant de 0,05 à 5% en poids, par rapport au poids total de la composition, et de préférence de 0,1 à 3%.

13. Composition selon l'une quelconque des revendications précédentes, caractérisée par le fait que le colorant direct est présent, sous forme de base ou salifiée, dans des proportions allant de 0,001 à 10% en poids, par rapport au poids total de la composition, et de préférence de 0,05 à 5%.

14. Composition selon l'une quelconque des revendications précédentes, caractérisée par le fait que le support cosmétiquement acceptable approprié pour la teinture est un support aqueux constitué par de l'eau et/ou des solvants organiques choisis parmi les alcools, les glycols et les éthers de glycol, dans des proportions comprises entre 0,5 et 20% en poids par rapport au poids total de la composition.

15. Utilisation d'un polymère réticulé tel que défini dans l'une quelconque des revendications 1 à 7, dans ou pour la fabrication d'une, composition de teinture directe pour cheveux comprenant au moins un colorant direct, pour améliorer la conservation du pouvoir tinctorial de ladite composition, en particulier après des stockages à basses températures.

16. Procédé pour améliorer la conservation du pouvoir tinctorial, en particulier après des stockages à basses températures, d'une composition de teinture contenant au moins un colorant direct, caractérisé par le fait qu'on ajoute à ladite composition, une quantité efficace d'un polymère réticulé tel que défini dans l'une quelconque des revendications 1 à 7.

17. Procédé de teinture des cheveux, par coloration directe, caractérisé par le fait qu'on applique une composition tinctoriale telle que définie à l'une quelconque des revendications 1 à 14 sur les cheveux secs ou humides, on laisse agir la composition, on rince les cheveux, éventuellement on les lave, puis on les rince à nouveau, enfin on les sèche.

18. Procédé de teinture des cheveux, par coloration directe, caractérisé par le fait qu'on applique une composition tinctoriale telle que définie à l'une quelconque des revendications 1 à 14 sur les cheveux secs ou humides, et qu'après avoir laissé agir la composition, on sèche les cheveux.

## Claims

1. Composition for dyeing the hair, of the type comprising, in a cosmetically acceptable support which is suitable for dyeing, at least one direct dye, characterized in that it also comprises at least one crosslinked polymer containing acrylic and/or acrylate units and acrylamide units.

2. Dye composition according to Claim 1, characterized in that the crosslinked polymer contains acrylate units and acrylamide units.

3. Dye composition according to Claim 2, characterized in that the crosslinked polymer is an ammonium acrylate/acrylamide crosslinked copolymer.

4. Dye composition according to Claim 2, characterized in that the crosslinked polymer is a sodium acrylate/acrylamide crosslinked copolymer.

5. Dye composition according to any one of the preceding claims, characterized in that the crosslinking agent is a compound containing olefinic polyunsaturation chosen from divinylbenzene, tetraallyloxyethane, methylenebisacrylamide, diallyl ether, polyallylpolyglyceryl ethers or allylic ethers of alcohols of the sugar series.

6. Composition according to Claims 1, 2, 3 and 5, characterized in that the crosslinked polymer is an ammonium acrylate/acrylamide crosslinked copolymer in a proportion of 95/5 by weight.

7. Composition according to Claim 6, characterized in that the copolymer is in the form of a water-in-oil emulsion consisting of 32% by weight of the said copolymer, 20% by weight of isoparaffin, 2% by weight of sorbitan sesquioleate 3% by weight of a hydrophilic ethoxylated derivative and 43% by weight of water.

8. Composition according to any one of the preceding claims, characterized in that the direct dye is a nitrobenzene dye of formula (I) below: 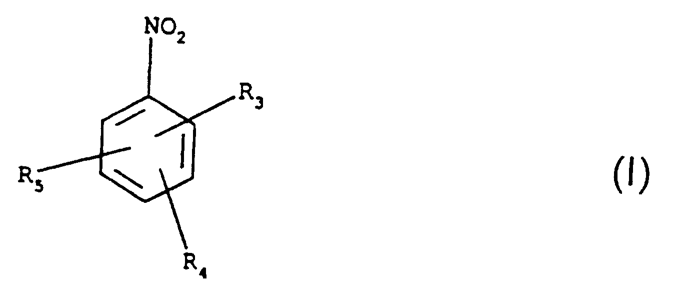 in which:
- R₃ denotes an NH₂ radical, an amino radical monosubstituted with an alkyl, monohydroxyalkyl, polyhydroxyalkyl or aminoalkyl radical, or an amino radical disubstituted with identical or different alkyl, mono- or polyhydroxyalkyl or aminoalkyl radicals,
- R₄ denotes hydrogen, hydroxyl, alkoxy, mono- or polyhydroxyalkyloxy, or the same meanings denoted above for R₃, except for the disubstituted amino radical,
- R₅ denotes hydrogen, alkyl, nitro or halogen,
it being understood that the alkyl and alkoxy radicals mentioned above are C₁-C₄ and that they can be linear or branched,
and the cosmetically acceptable salts of these compounds.

9. Composition according to Claims 1 to 7, characterized in that the direct dye is an anthraquinone dye of formula (II) below: 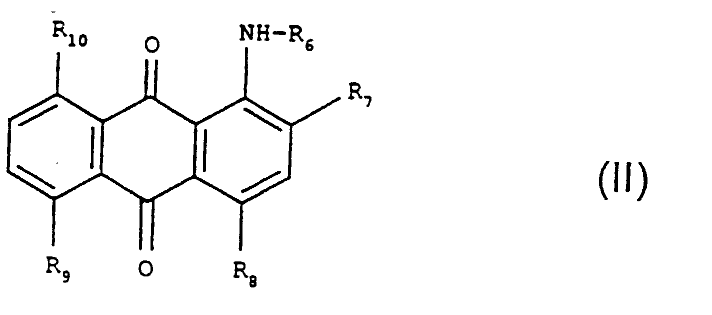 in which
- R₆ denotes hydrogen or a monohydroxyalkyl or polyhydroxyalkyl radical,
- R₇ denotes hydrogen or an alkyl or alkoxy radical,
- R₈ denotes hydrogen or a hydroxyl, amino, monohydroxyalkylamino or polyhydroxyalkylamino radical,
- R₉ and R₁₀, which may be identical or different, are hydrogen, hydroxyl or amino,
it being understood that the alkyl and alkoxy radicals mentioned above are C₁-C₄ and that they can be linear or branched,
and the cosmetically acceptable salts of these compounds.

10. Composition according to Claims 1 to 7, characterized in that the direct dye is an azo dye of formula (III) below: 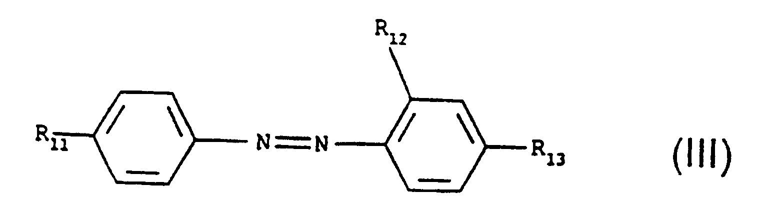 in which:
- R₁₁ denotes a nitro or amino radical or an amino radical mono- or disubstituted with alkyls,
- R₁₂ denotes hydrogen or an alkyl radical,
- R₁₃ denotes an amino radical or an amino radical mono- or disubstituted with monohydroxyalkyls, it being understood that the alkyl and alkoxy radicals mentioned above are C₁-C₄ and that they can be linear or branched,
and the cosmetically acceptable salts of these compounds.

11. Composition according to Claims 8 to 10, characterized in that the cosmetically acceptable salts are hydrochlorides, hydrobromides and sulphates.

12. Composition according to any one of the preceding claims, characterized in that the crosslinked polymer is present, calculated in terms of active material, in proportions ranging from 0.05 to 5% by weight, relative to the total weight of the composition, and preferably from 0.1 to 3%.

13. Composition according to any one of the preceding claims, characterized in that the direct dye is present, in salified or base form, in proportions ranging from 0.001 to 10% by weight, relative to the total weight of the composition, and preferably from 0.05 to 5%.

14. Composition according to any one of the preceding claims, characterized in that the cosmetically acceptable support which is suitable for dyeing is an aqueous support consisting of water and/or organic solvents chosen from alcohols, glycols and glycol ethers, in proportions of between 0.5 and 20% by weight relative to the total weight of the composition.

15. Use of a crosslinked polymer as defined in any one of Claims 1 to 7, in, or for the manufacture of, a direct dye composition for the hair, comprising at least one direct dye, in order to improve the conservation of the dyeing power of the said composition, in particular after storage at low temperatures.

16. Process for improving the conservation of the dyeing power, in particular after storage at low temperatures, of a dye composition containing at least one direct dye, characterized in that an effective amount of a crosslinked polymer as defined in any one of Claims 1 to 7 is added to the said composition.

17. Process for dyeing the hair by direct dyeing, characterized in that a dye composition as defined in any one of Claims 1 to 14 is applied to wet or dry hair, the composition is left to act, the hair is rinsed, it is optionally washed, it is then rinsed again and, lastly, it is dried.

18. Process for dyeing the hair by direct dyeing, characterized in that a dye composition as defined in any one of Claims 1 to 14 is applied to wet or dry hair and, after the composition has been left to act, the hair is dried.

## Patentansprüche

1. Zusammensetzung zum Färben von Haaren, die in einem kosmetisch akzeptablen und zum Färben geeigneten Träger mindestens einen Direktfarbstoff enthält, dadurch gekennzeichnet, dass sie ferner mindestens ein vernetztes Polymer mit Acryleinheiten und/oder Acrylateinheiten und mit Acrylamideinheiten aufweist.

2. Zusammensetzung zum Färben nach Anspruch 1, dadurch gekennzeichnet, dass das vernetzte Polymer ein Polymer mit Acrylateinheiten und mit Acrylamideinheiten ist.

3. Zusammensetzung zum Färben nach Anspruch 2, dadurch gekennzeichnet, dass das vernetzte Polymer ein vernetztes Ammoniumacrylat/Acrylamid-Copolymer ist.

4. Zusammensetzung zum Färben nach Anspruch 2, dadurch gekennzeichnet, dass das vernetzte Polymer ein vernetztes Natriumacrylat/Acrylamid-Copolymer ist.

5. Zusammensetzung zum Färben nach einem der vorhergehenden Ansprüche, dadurch gekennzeichnet, dass das Vernetzungsmittel eine mehrfach olefinisch ungesättigte Verbindung ist, die unter Divinylbenzol, Tetraallyloxyethan, Methylen-bis-acrylamid, Diallylether, Polyallylpolyglycerylethem oder Allylethern von Alkoholen aus der Zuckerreihe ausgewählt ist.

6. Zusammensetzung nach den Ansprüchen 1, 2, 3 und 5, dadurch gekennzeichnet, dass das vernetzte Polymer ein vernetztes Ammoniumacrylat/Acrylamid-Copolymer mit einem Gewichtsverhältnis von 95/5 ist.

7. Zusammensetzung nach Anspruch 6, dadurch gekennzeichnet, dass das Copolymer in Form einer Wasser-in-Öl-Emulsion vorliegt, die aus 32 Gew.-% dieses Copolymers, 20 Gew.-% Isoparaffin, 3 Gew.-% Sorbitansesquioleat, 2 Gew.-% eines hydrophilen ethoxylierten Derivats und 43 Gew.-% Wasser besteht.

8. Zusammensetzung nach einem der vorhergehenden Ansprüche, dadurch gekennzeichnet, dass der Direktfarbstoff ein nitrierter Benzolfarbstoff der folgenden Formel (I): 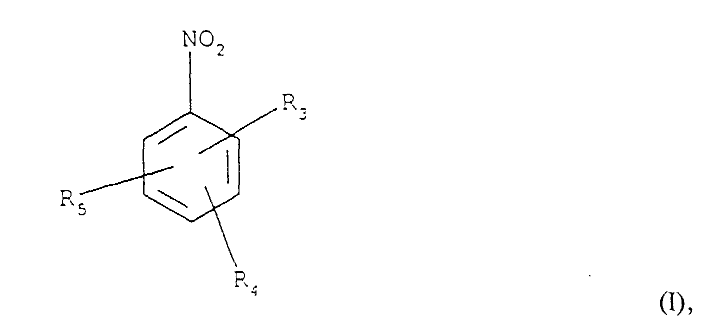 worin bedeuten:
- R₃ die Gruppe NH₂, eine einfach mit einer Alkylgruppe, einer Monohydroxyalkylgruppe, einer Polyhydroxyalkylgruppe oder einer Aminoalkylgruppe substituierte Aminogruppe oder eine zweifach mit Alkylgruppen, Monohydroxyalkylgruppen, Polyhydroxyalkylgruppen oder Aminoalkylgruppen, die identisch oder voneinander verschieden sind, substituierte Aminogruppe,
- R₄ Wasserstoff, Hydroxy, Alkoxy, Monohydroxyalkyloxy, Polyhydroxyalkyloxy oder die oben für R₃ genannten Gruppen mit Ausnahme der zweifach substituierten Aminogruppe und
- R₅ Wasserstoff, Alkyl, Nitro oder Halogen,
mit der Maßgabe, dass die oben genannten Alkylgruppen und Alkoxygruppen 1 bis 4 Kohlenstoffatome aufweisen und dass sie geradkettig oder verzweigt vorliegen können,
oder ein kosmetisch akzeptables Salz dieser Verbindungen ist.

9. Zusammensetzung nach den Ansprüchen 1 bis 7, dadurch gekennzeichnet, dass der Direktfarbstoff ein Anthrachinonfarbstoff der folgenden Formel (II): 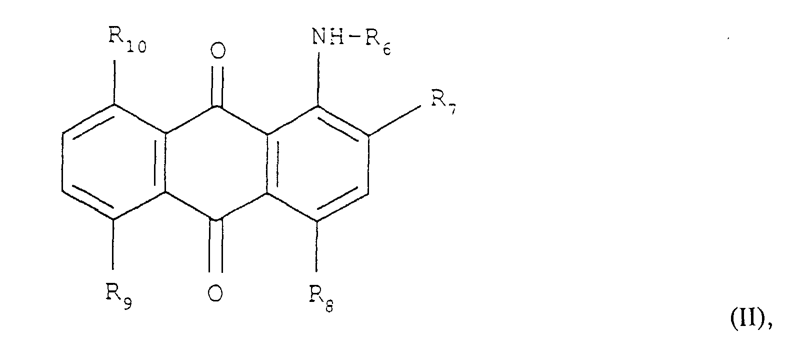 worin bedeuten:
- R₆ Wasserstoff, eine Monohydroxyalkylgruppe oder eine Polyhydroxyalkylgruppe,
- R₇ Wasserstoff, eine Alkylgruppe oder eine Alkoxygruppe,
- R₈ Wasserstoff, eine Hydroxygruppe, eine Aminogruppe, eine Monohydroxyalkylaminogruppe oder eine Polyhydroxyalkylaminogruppe und
- R₉ und R₁₀, die identisch oder voneinander verschieden sind, Wasserstoff, Hydroxy oder Amino,
mit der Maßgabe, dass die oben genannten Alkylgruppen und Alkoxygruppen 1 bis 4 Kohlenstoffatome aufweisen und dass sie geradkettig oder verzweigt vorliegen können,
oder ein kosmetisch akzeptables Salz dieser Verbindungen ist.

10. Zusammensetzung nach den Ansprüchen 1 bis 7, dadurch gekennzeichnet, dass der Direktfarbstoff ein Azofarbstoff der folgenden Formel (III): 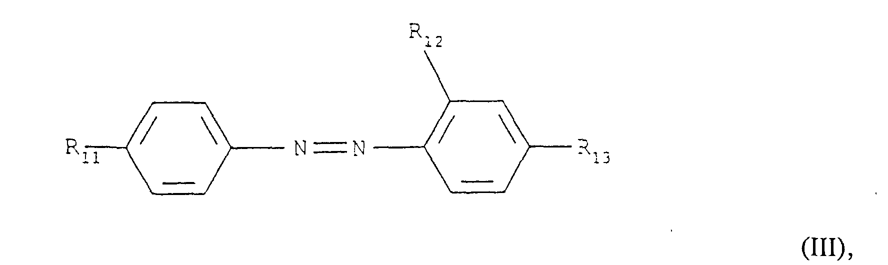 worin bedeuten:
- R₁₁ eine Nitrogruppe, eine Aminogruppe oder eine einfach oder zweifach mit Alkylgruppen substituierte Aminogruppe,
- R₁₂ Wasserstoff oder eine Alkylgruppe und
- R₁₃ eine Aminogruppe oder eine einfach oder zweifach mit Monohydroxyalkylgruppen substituierte Aminogruppe,
mit der Maßgabe, dass die oben genannten Alkylgruppen und Alkoxygruppen 1 bis 4 Kohlenstoffatome aufweisen und dass sie geradkettig oder verzweigt vorliegen können,
oder ein kosmetisch akzeptables Salz dieser Verbindungen ist.

11. Zusammensetzung nach den Ansprüchen 8 bis 10, dadurch gekennzeichnet, dass die kosmetisch akzeptablen Salze Chlorhydrate, Bromhydrate und Sulfate sind.

12. Zusammensetzung nach einem der vorhergehenden Ansprüche, dadurch gekennzeichnet, dass das vernetzte Polymer in Mengenanteilen (berechnet als Wirkstoffgehalt) im Bereich von 0,05 bis 5 Gew.-% und vorzugsweise von 0,1 bis 3 Gew.-%, bezogen auf das Gesamtgewicht der Zusammensetzung, vorliegt.

13. Zusammensetzung nach einem der vorhergehenden Ansprüche, dadurch gekennzeichnet, dass der Direktfarbstoff als solcher oder in Form eines Salzes in Mengenanteilen im Bereich von 0,001 bis 10 Gew.-% und vorzugsweise von 0,05 bis 5 Gew.-%, bezogen auf das Gesamtgewicht der Zusammensetzung, vorliegt.

14. Zusammensetzung nach einem der vorhergehenden Ansprüche, dadurch gekennzeichnet, dass der kosmetisch akzeptable und zum Färben geeignete Träger ein wässeriger Träger ist, der aus Wasser und/oder organischen Lösemitteln besteht, die unter Alkoholen, Glykolen und Glykolethern ausgewählt sind und in Mengenanteilen im Bereich von 0,5 bis 20 Gew.-%, bezogen auf das Gesamtgewicht der Zusammensetzung, vorliegen.

15. Verwendung eines in einem der Ansprüche 1 bis 7 definierten vernetzten Polymers in einer Zusammensetzung zur Direktfärbung von Haaren, die mindestens einen Direktfarbstoff enthält, oder zu ihrer Herstellung, damit das Färbevermögen der Zusammensetzung insbesondere nach Aufbewahrung bei niedrigen Temperaturen besser erhalten bleibt.

16. Verfahren, um das Färbevermögen einer Färbemittelzusammensetzung, die mindestens einen Direktfarbstoff enthält, insbesondere nach Aufbewahrung bei niedrigen Temperaturen besser zu bewahren, dadurch gekennzeichnet, dass eine wirksame Menge eines in einem der Ansprüche 1 bis 7 definierten vernetzten Polymers zu der Zusammensetzung gegeben wird.

17. Verfahren zum Färben von Haaren durch Direktfärbung, dadurch gekennzeichnet, dass eine in einem der Ansprüche 1 bis 14 definierte Färbemittelzusammensetzung auf das trockene oder feuchte Haar aufgebracht wird, die Zusammensetzung dann einwirken gelassen wird, das Haar gespült, anschließend gegebenenfalls gewaschen und erneut gespült und schließlich getrocknet wird.

18. Verfahren zum Färben von Haaren durch Direktfärbung, dadurch gekennzeichnet, dass eine in einem der Ansprüche 1 bis 14 definierte Färbemittelzusammensetzung auf das trockene oder feuchte Haar aufgebracht wird, die Zusammensetzung dann einwirken gelassen wird und das Haar anschließend getrocknet wird.
